# EUROPEAN PATENT APPLICATION

(11) **EP 4 809 939 A1**
(43) Date of publication of application: **23.09.2026**
(21) Application number: 24890581.2
(22) Date of filing: 08.11.2024
(51) Int. Cl.: A61B 17/11, A61F 2/06

(54) **BLOOD VESSEL CONNECTION ASSEMBLY**

(30) Priority: 13.11.2023 CN 202311511736
(71) Applicant: Cheetah Technology Co. Ltd Shenzhen, Shenzhen, Guangdong 518107 (CN)
(72) Inventor: GAO, Feng, Shenzhen, Guangdong 518055 (CN)
(74) Representative: CAPRI
(86) International application number: PCT/CN2024/130810
(87) International publication number: WO 2025/103225

(57) **Abstract**

A blood vessel connection assembly, comprising an artificial blood vessel (1) and a support tube (2), wherein the support tube (2) is sleeved on and connected to the artificial blood vessel (1), and is fixed to the end of the artificial blood vessel (1). The blood vessel connection assembly enables the artificial blood vessel (1) and the support tube (2) to be fixed to each other to form one piece. During an artificial blood vessel surgery, the whole structure can be directly installed, thus reducing the connection operation between the artificial blood vessel (1) and the support tube (2) during surgery, thereby effectively shortening the surgery duration and improving the surgery efficiency.

## Description

The present application claims the priority to Chinese Patent Application No. 202311511736.0, titled "BLOOD VESSEL CONNECTION ASSEMBLY", filed on November 13, 2023 with the China National Intellectual Property Administration, which is incorporated herein by reference in its entirety.

### FIELD

The present application relates to the technical field of implantable medical devices, and in particular to a blood vessel connection assembly.

### BACKGROUND

In the conventional technology, an artificial blood vessel is generally of a straight cylindrical structure, and is radially supported by a support tube. The artificial blood vessel is sleeved over a human blood vessel, and finally secured by binding with a binding thread.

During surgery, a clinician needs to first fix the artificial blood vessel and the support tube, then sleeve the artificial blood vessel over the human blood vessel, fix the artificial blood vessel and the human blood vessel in place, and finally secure them with a binding thread. This surgical procedure is time-consuming. Since the surgical time is directly correlated with trauma, a prolonged vascular suturing time may extend the cardiac arrest time, causing physical damage to the patient and posing significant safety risks.

Therefore, in the process of performing artificial blood vessel surgery, how to shorten the surgical time, improve surgical efficiency, and avoid physical damage to the patient due to a prolonged surgical procedure are technical issues to be addressed urgently by those skilled in the art.

### SUMMARY

An object of the present application is to provide a blood vessel connection assembly, which, during artificial blood vessel surgery, can shorten surgical time, improve surgical efficiency, and avoid physical damage to the patient due to a prolonged surgical procedure.

To address the above technical issues, a blood vessel connection assembly is provided according to the present application, including an artificial blood vessel and a support tube. The support tube is sleeved onto the artificial blood vessel and is fixed to an end portion of the artificial blood vessel.

Optionally, there are two support tubes, and the two support tubes are respectively fixed to two ends of the artificial blood vessel.

Optionally, the blood vessel connection assembly further includes at least two connection portions, with each connection portion including a connecting thread. The connecting thread passes through the artificial blood vessel and/or the support tube, and both ends of the connecting thread are located outside the artificial blood vessel and the support tube.

Optionally, the connection portion further includes a suture needle provided on the connecting thread.

Optionally, there are three connection portions, and the connection portions are evenly spaced along a circumferential direction of the support tube.

Optionally, a marking line is further provided on an outer wall of the support tube or an outer wall of the artificial blood vessel.

Optionally, an annular mounting cavity is provided at the end portion of the artificial blood vessel. The annular mounting cavity is configured for mounting the support tube, and at least one side end of the annular mounting cavity is closed so as to isolate the support tube from blood flowing through the artificial blood vessel.

Optionally, the artificial blood vessel includes a first tube body and a second tube body sleeved over an end portion of the first tube body. The annular mounting cavity is enclosed and defined between the first tube body and the second tube body, and an end portion of the annular mounting cavity is circumferentially closed.

Optionally, two ends of the second tube body are configured to be respectively located on two sides of a binding position. The binding position is a position where the blood vessel connection assembly is bound to a human blood vessel with a binding thread.

Optionally, the end portion of the first tube body is turned outward and extends axially to form the second tube body.

Optionally, the artificial blood vessel and the support tube are fixed by suturing; or,
multiple spinous protrusions are further provided on the outer wall of the support tube at intervals, with the spinous protrusions passing through the second tube body so as to be fixed to the artificial blood vessel.

Optionally, a through hole is provided on a side wall of the support tube.

Optionally, the support tube has a curved axis.

The blood vessel connection assembly provided according to the present application has the following beneficial effects compared to the conventional technology.

The support tube and the end portion of the artificial blood vessel are fixedly connected. Such an arrangement ensures the stability of the blood vessel connection assembly. During surgery, when sleeving the blood vessel connection assembly onto the human blood vessel, there is no need to perform a fixing operation between the artificial blood vessel and the support tube, thereby simplifying the surgical operation, reducing surgical time, improving surgical efficiency, and lowering safety risks.

Moreover, directly securing the artificial blood vessel and the support tube also ensures that the fixation between the two meets the needs of different individuals, resulting in a wider scope of application, and avoids influences on the patient due to different fixa tion methods or degrees between the artificial blood vessel and the support tube during surgery caused by different techniques of clinicians.

### BRIEF DESCRIPTION OF THE DRAWINGS

FIG. 1 is a cross-sectional view of a blood vessel connection assembly provided according to an embodiment of the present application;
FIG. 2 is a partial enlarged view of FIG. 1; and
FIG. 3 is a cross-sectional view showing the blood vessel connection assembly sleeved onto a human blood vessel.

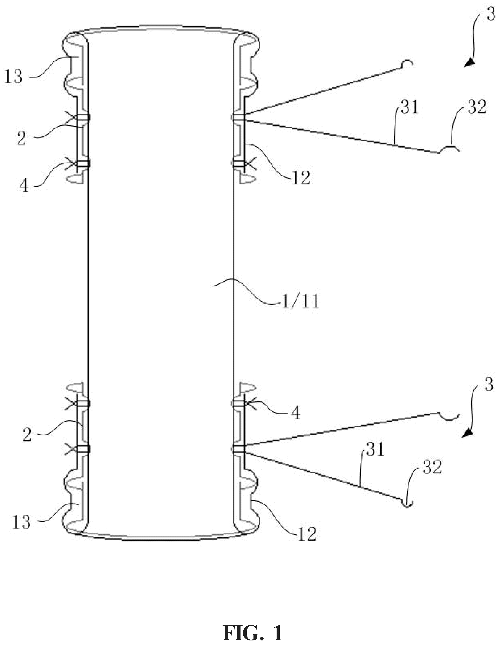

Reference numerals in FIGS. 1 to 3 are as follows:
1 artificial blood vessel, 11 first tube body, 12 second tube body, 13 annular mounting cavity;
2 support tube, 21 through hole, 22 annular protrusion;
3 connection portion, 31 connecting thread, 32 suture needle;
4 fixing point;
5 human blood vessel.

### DETAILED DESCRIPTION OF THE EMBODIMENTS

In order to enable those skilled in the art to better understand the technical solutions of the present application, the present application will be further described in detail below with reference to the accompanying drawings and specific embodiments.

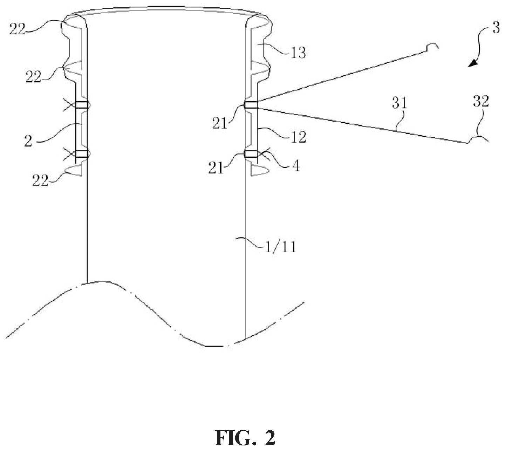

A blood vessel connection assembly is provided according to an embodiment of the present application, configured for connecting a human blood vessel 5. Specifically, as shown in FIGS. 1 and 2, the blood vessel connection assembly includes an artificial blood vessel 1 and a support tube 2. The support tube 2 is sleeved onto the artificial blood vessel 1 and is fixed to an end portion of the artificial blood vessel 1. The support tube 2 is typically a metal tube (such as a nickel-titanium alloy tube), capable of providing radial support to the artificial blood vessel 1.

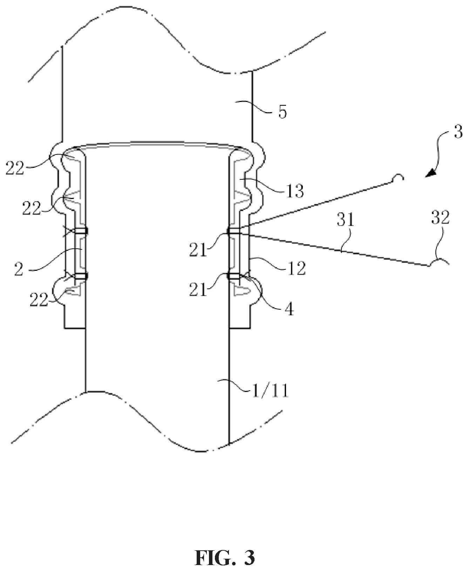

As shown in FIG. 3, when connecting the human blood vessel 5 through the blood vessel connection assembly, an end portion of the blood vessel connection assembly is sleeved onto the human blood vessel 5, and secured by binding with a binding thread at a binding position. The binding position may be radially supported by the support tube 2 to ensure connection stability.

In the blood vessel connection assembly provided in this embodiment, the support tube 2 and the end portion of the artificial blood vessel 1 are fixedly connected. Such an arrangement ensures the stability of the blood vessel connection assembly. During surgery, when sleeving the blood vessel connection assembly onto the human blood vessel 5, there is no need to perform a fixing operation between the artificial blood vessel 1 and the support tube 2, thereby simplifying the surgical operation, reducing surgical time, improving surgical efficiency, and lowering safety risks.

Moreover, directly securing the artificial blood vessel 1 and the support tube 2 also ensures that the fixation between the two meets the needs of different individuals, resulting in a wider scope of application, and avoids influences on the patient due to different fixation methods or degrees between the artificial blood vessel 1 and the support tube 2 during surgery caused by different techniques of clinicians.

As shown in FIGS. 1 and 2, there are two support tubes 2, and the two support tubes 2 are respectively sleeved onto and fixed to two ends of the artificial blood vessel 1. When connecting the human blood vessel 5 through the blood vessel connection assembly, the two ends of the blood vessel connection assembly are respectively sleeved onto and fixed to the human blood vessel 5.

As shown in FIGS. 1 to 3, the blood vessel connection assembly is further provided with a connection portion 3. The connection portion 3 includes a connecting thread 31 and a suture needle 32. The connecting thread 31 passes through the artificial blood vessel 1 and the support tube 2, and both ends of the connecting thread 31 are located outside the artificial blood vessel 1 and the support tube 2 and are threaded with the suture needles 32 respectively.

During surgery, after aligning the angle between the end portion of the blood vessel connection assembly and the human blood vessel 5, the suture needles 32 at the two ends of the connecting thread 31 are respectively passed from inside to outside through different positions of the human blood vessel 5. Then, the human blood vessel 5 is sleeved onto the blood vessel connection assembly, and the two ends of the connecting thread 31 are tied and sutured, so that the human blood vessel 5 and the blood vessel connection assembly are stably connected. Finally, the human blood vessel 5 and the blood vessel connection assembly are secured by binding with a binding thread.

That is to say, in this embodiment, the artificial blood vessel 1, the support tube 2, and the connection portion 3 are connected as an integral whole. During surgery, the mounting and suturing operations with the human blood vessel 5 may be directly performed. Compared with the conventional technology where the artificial blood vessel 1 and the support tube 2 are provided as separate structures, and the artificial blood vessel 1 and the support tube 2 are sutured and fixed on site during surgery, and then the blood vessel connection assembly and the human blood vessel 5 are sutured on site through the connecting thread 31 and the suture needle 32, the solution of the present application reduces the time for securing the artificial blood vessel 1 and the support tube 2, reduces the time for passing the connecting thread 31 through the artificial blood vessel 1 and the support tube 2 on site, effectively improves surgical efficiency, lowers the probability of physical damage to the patient due to prolonged vascular suturing time, and lowers safety risks.

Moreover, since circumferential positions of the connection portions 3 on the support tube 2 in the blood vessel connection assembly are predetermined, during surgery, it is only necessary to ensure the alignment angle between the blood vessel connection assembly and the human blood vessel 5 to ensure the suturing positions of the connection portions 3 with respect to the human blood vessel 5. When suturing the human blood vessel 5, there is no need to re-identify the suturing positions, further reducing the surgical suturing time, while also lowering the requirements for the suturing skills of the clinicians, ensuring that the suturing positions and morphology meet requirements, and lowering safety risks.

Of course, the connection portion 3 may only include the connecting thread 31. During surgery, the suture needle 32 is then connected to the connecting thread 31 before suturing. When the ends of the connecting thread 31 are directly connected with the suture needles 32, the surgical duration may be further reduced.

Furthermore, in this embodiment, the connecting thread 31 may only pass through the artificial blood vessel 1 or only pass through the support tube 2. That is, the connecting thread 31 may be used for suturing connection between the artificial blood vessel 1 and the human blood vessel 5, or between the support ring 2 and the human blood vessel 5. However, when the connecting thread 31 passes through the artificial blood vessel 1, the support ring 2, and the human blood vessel 5 to achieve suturing connection, the stability of the suturing connection may be further enhanced.

Further, in this embodiment, there are three connection portions 3, and the connection portions 3 are evenly spaced along a circumferential direction of the support tube 2. When the human aorta is connected through this blood vessel connection assembly, the aorta is provided with three aortic valves along its circumference. Generally, from a cross-sectional view, these three aortic valves are approximately evenly spaced circumferentially, with commissure points between every adjacent aortic valves. During mounting, the connecting thread 31 may pass through these commissure points.

In this embodiment, the positions of the three connection portions 3 in the axial direction of the support tube 2 are not limited. For example, the three connection portions 3 may be arranged in the same cross-section in the axial direction of the support tube 2, or may be arranged in different cross-sections in the axial direction of the support tube 2.

Of course, in this embodiment, the number of the connection portions 3 is not limited. For example, there may be two, four, or more connection portions 3, which may be determined based on the application requirements of the blood vessel connection assembly.

In this embodiment, a marking line is further provided on an outer surface of the support tube 2 or the artificial blood vessel 1. The marking line can ensure the mounting angle between the blood vessel connection assembly and the human blood vessel 5 during surgery, maximizing the close fit between the artificial blood vessel 1 and the inner wall of the human blood vessel 5 via the support tube 2, and avoiding blood seepage.

In this embodiment, annular mounting cavities 13 are further respectively provided at the two ends of the artificial blood vessel 1. The annular mounting cavity 13 is configured for mounting the support tube 2. The annular mounting cavity 13 has two side ends, at least one of which is closed, so that the support tube 2 inside is isolated from blood flowing through the artificial blood vessel 1, thereby avoiding the formation of blood clots due to direct contact of the metal support tube 2 with blood, and also avoiding turbulent flow or other disturbed blood flow caused by contact between the edge of the support tube 2 and high-speed blood flow, thus ensuring smooth blood flow.

As shown in FIGS. 2 and 3, the artificial blood vessel 1 includes a first tube body 11 and two second tube bodies 12. The two second tube bodies 12 are respectively located at two ends of the first tube body 11, and the second tube bodies 12 are sleeved over the first tube body 11. Each annular mounting cavity 13 is enclosed and defined between the first tube body 11 and the second tube body 12, and a side end of the annular mounting cavity 13 away from the other annular mounting cavity 13 is circumferentially closed.

Of course, in this embodiment, the structure of the annular mounting cavity 13 is not limited. Furthermore, the inner wall and outer wall of the support tube 2 may be respectively wrapped with coverings, the material of which may be the same as that of the artificial blood vessel 1, and then the wrapped support tube 2 is sleeved onto and sutured to the artificial blood vessel 1.

When the annular mounting cavity 13 is enclosed and defined between the first tube body 11 and the second tube body 12, the overall structure can be simplified, the cost can be reduced, and the overall thickness of the end portion of the blood vessel connection assembly can be reduced, thus avoiding obstruction to blood flow.

The first tube body 11 is located inside the support tube 2, and the second tube body 12 is located outside the support tube 2. Blood flows inside the first tube body 11. Thus, compared with a solution where the support tube 2 is arranged inside the artificial blood vessel 1, this solution can avoid interference of the support tube 2 with blood flow. An inner diameter of the support tube 2 matches an outer diameter of the first tube body 11, so that the inner wall surface of the first tube body 11 is smooth, avoiding the influence of wrinkles on the inner wall surface of the first tube body 11 on blood flow.

The two axial ends of the second tube body 12 are respectively located on two sides of the binding position. With such an arrangement, the side end of one annular mounting cavity 13 facing the other annular mounting cavity 13 may be an open structure, ensuring that the support tube 2 is separated from the blood flowing inside the blood vessel, while further simplifying the manufacturing process of the blood vessel connection assembly. Moreover, when assembling the blood vessel connection assembly, the support tube 2 can be mounted into the annular mounting cavity 13 through the open structure, making the operation more convenient.

In this embodiment, the end portion of the first tube body 11 is turned outward and extends axially to form the second tube body 12. That is, the first tube body 11 and the second tube body 12 are an integral structure formed by turning the end portion of the first tube body 11. This can simplify the overall structure of the artificial blood vessel 1 while ensuring the sealing of the end portion of the annular mounting cavity 13.

Of course, when manufacturing the artificial blood vessel 1, the second tube bodies 12 may be directly formed on the outer wall of the first tube body 11, such that the end of one second tube body 12 away from the other second tube body 12 is circumferentially attached and sealed to the end portion or the outer peripheral wall of the first tube body 11.

Forming the second tube body 12 by turning the end portion of the first tube body 11 simplifies the overall structure, simplifies the manufacturing process, reduces costs, and allows direct use of the existing artificial blood vessel 1 from the conventional technology, providing good flexibility.

Specifically, the method for securing the artificial blood vessel 1 and the support tube 2 is not limited. As shown in FIGS. 2 and 3, there may be multiple fixing points 4 between the artificial blood vessel 1 and the support tube 2 to ensure the stability of the fixation therebetween. Each fixing point 4 may be formed by suturing. Alternatively, the support tube 2 may be provided with spinous protrusions on its outer wall that can penetrate the second tube body 12 to restrict the support tube 2 from detaching from the second tube body 12, thereby achieving fixation with the artificial blood vessel 1. This can further simplify the operation for securing the support tube 2 and the artificial blood vessel 1.

As shown in FIGS. 2 and 3, a through hole 21 is further provided on the side wall of the support tube 2. The connecting thread 31 of the connection portion 3 passes through the through hole 21. Moreover, when the fixing point 4 between the artificial blood vessel 1 and the support tube 2 is formed by suturing, the support tube 2 is also provided with a corresponding through hole 21 at the fixing point 4 to facilitate the suturing operation.

To further facilitate the suturing operation, a guide surface may be further provided at the end of the through hole 21 on the side wall of the support tube 2. The guide surface may be either an inclined surface or an arc surface. Meanwhile, the provision of the through hole 21 may also reduce the overall weight of the support tube 2.

Specifically, as shown in FIG. 2, the through holes 21 are distributed on the side wall of the support tube 2 close to the other support tube 2. During subsequent binding with a binding thread, the binding position is generally located at a middle portion of the support tube 2 and on a side of the support tube 2 away from the other support tube 2. The through holes 21 are arranged staggered from the binding position, which ensures that the support tube 2, during the subsequent binding with the binding thread, can provide sufficient radial support, thereby ensuring binding stability.

In this embodiment, there is no limitation on the quantity, size, or the like, of the through holes 21, as long as the through holes 21 are provided at positions requiring suturing.

The axis of the support tube 2 may be a straight line or a curved line. When the axis of the support tube 2 is a curved line, the curvature of the curved line may be designed based on the curvature of the human blood vessel 5 at that location.

Moreover, when the axis of the support tube 2 is a curved line, after the human blood vessel 5 and the blood vessel connection assembly are connected in a sleeved manner, the support tube 2 can provide radial support and make the outer wall of the blood vessel connection assembly tightly fit with the inner wall of the human blood vessel 5, avoiding blood seepage, while also ensuring the support stability of the support tube 2, and facilitating subsequent binding operations.

Annular protrusions 22 are respectively provided at two ends of the support tube 2, which restrict the position of the binding thread, preventing the binding thread from detaching from the end portions of the support tube 2, and ensuring the binding stability of the binding thread.

Specifically, three annular protrusions 22 in total are provided on the outer wall of the support tube 2. Two of the annular protrusions 22 are respectively provided at the two end portions of the support tube 2, and, a third annular protrusion 22 is provided on the side of the support tube 2 away from the other support tube 2, so that on the side of the binding position close to the end portion of the artificial blood vessel 1, the binding thread is limited by the two spaced-apart annular protrusions 22, providing good stability.

In addition, a branch blood vessel (not shown in the figures) may be further provided at the middle portion of the artificial blood vessel 1 in a communicating manner. The branch blood vessel is communicated with the first tube body 11 and is generally arranged radially outward of the artificial blood vessel 1. The quantity, position, and the like, of the branch blood vessels may be set based on the actual application position and requirements of the blood vessel connection assembly, which are not specifically limited here.

In this embodiment, the cross-section of the support tube 2 may be circular, elliptical, polygonal in shape, or have other irregular shapes, which is not specifically limited herein.

The above are only preferred embodiments of the present application. It should be noted that for those skilled in the art, several improvements and modifications can be made without departing from the principle of the present application, and these improvements and modifications should also be regarded as the scope of protection of the present application.

## Claims

1. A blood vessel connection assembly, comprising an artificial blood vessel (1) and a support tube (2), wherein
the support tube (2) is sleeved onto the artificial blood vessel (1) and is fixed to an end portion of the artificial blood vessel (1).

2. The blood vessel connection assembly according to claim 1, wherein there are two support tubes (2), and the two support tubes (2) are respectively fixed to two ends of the artificial blood vessel (1).

3. The blood vessel connection assembly according to claim 1, further comprising at least two connection portions (3), wherein
each connection portion (3) comprises a connecting thread (31), the connecting thread (31) passes through the artificial blood vessel (1) and/or the support tube (2), and both ends of the connecting thread (31) are located outside the artificial blood vessel (1) and the support tube (2).

4. The blood vessel connection assembly according to claim 3, wherein the connection portion (3) further comprises a suture needle (32) provided on the connecting thread (31).

5. The blood vessel connection assembly according to claim 4, wherein there are three connection portions (3), and the connection portions (3) are evenly spaced along a circumferential direction of the support tube (2).

6. The blood vessel connection assembly according to claim 5, wherein a marking line is further provided on an outer wall of the support tube (2) or an outer wall of the artificial blood vessel (1).

7. The blood vessel connection assembly according to any one of claims 1 to 6, wherein an annular mounting cavity (13) is provided at the end portion of the artificial blood vessel (1), wherein the annular mounting cavity (13) is configured for mounting the support tube (2), and at least one side end of the annular mounting cavity (13) is closed so as to isolate the support tube (2) from blood flowing through the artificial blood vessel (1).

8. The blood vessel connection assembly according to claim 7, wherein the artificial blood vessel (1) comprises a first tube body (11) and a second tube body (12) sleeved over an end portion of the first tube body (11), the annular mounting cavity (13) is enclosed and defined between the first tube body (11) and the second tube body (12), and an end portion of the annular mounting cavity (13) is circumferentially closed.

9. The blood vessel connection assembly according to claim 8, wherein two ends of the second tube body (12) are configured to be respectively located on two sides of a binding position, the binding position being a position where the blood vessel connection assembly is bound to a human blood vessel (5) with a binding thread.

10. The blood vessel connection assembly according to claim 8, wherein the end portion of the first tube body (11) is turned outward and extends axially to form the second tube body (12).

11. The blood vessel connection assembly according to claim 8, wherein the artificial blood vessel (1) and the support tube (2) are fixed by suturing; or,
a plurality of spinous protrusions are further provided on the outer wall of the support tube (2) at intervals, with the spinous protrusions passing through the second tube body (12) so as to be fixed to the artificial blood vessel (1).

12. The blood vessel connection assembly according to any one of claims 1 to 6, wherein a through hole (21) is provided on a side wall of the support tube (2).

13. The blood vessel connection assembly according to any one of claims 1 to 6, wherein the support tube (2) has a curved axis.
